# EUROPÄISCHE PATENTANMELDUNG

(11) **EP 1 136 040 A2**
(43) Veröffentlichungstag der Anmeldung: **26.09.2001**
(21) Anmeldenummer: 01810211.1
(22) Anmeldetag: 01.03.2001
(51) Int. Cl.: A61B 17/02, A61F 9/007

(54) **Operationshaken für die Augenchirurgie**

(30) Priorität: 21.03.2000 US 528972
(71) Anmelder: GRIESHABER & CO. AG SCHAFFHAUSEN, CH-8203 Schaffhausen (CH)
(72) Erfinder: Grieshaber, Hans R., 8200 Schaffhausen (CH); Maag, Werner, 8750 Glarus (CH)
(74) Vertreter: Althoff, Gerhard

(57) **Zusammenfassung**

Es wird ein Operationshaken zum Zurückziehen der Regenbogenhaut (IRIS) beim Eingriff am Auge eines Lebewesens vorgeschlagen, welcher mindestens einen länglichen und an einem Ende zum Zurückziehen der Regenbogenhaut mit einem Einhängeteil versehenen Führungskörper sowie ein in axialer Richtung daran verschiebbar angeordnetes Fixierelement umfasst. Das Fixierelement (20;30) besteht beispielsweise aus einer optisch signalfarben eingefärbten Kautschukmischung, vorzugsweise aus einer Silikon-Kautschukmasse.

Bei dem Operationshaken (15;25) ist mindestens der in die Vorderkammer (V) des Auges (10) einführbare Führungskörper flexibel ausgebildet und aus einem in Bezug auf die Farbe der Regenbogenhaut (3) kontrastfarben eingefärbtem polymeren Material, beispielsweise aus thermoplastischem Material wie Polyamid oder dergleichen hergestellt.

## Beschreibung

Die Erfindung bezieht sich auf einen Operationshaken für die Augenchirurgie mit mindestens einem länglichen Führungskörper und daran angeordnetem Einhängeteil zum örtlichen Zurückziehen der Regenbogenhaut sowie einem verschiebbar an dem Führungskörper angeordneten Fixierelement zur Positionierung an der äusseren Kontur des Auges.

Bei speziellen operativen Eingriffen im vorderen und/oder hinteren Augenabschnitt ist es erforderlich ein ausreichend grosses und während des operativen Eingriffs unverändert bestehenbleibendes Sichtfeld für den Operateur zu schaffen. Das erforderliche Sichtfeld beziehungsweise die Vergrösserung der Pupillenöffnung kann beispielsweise medikamentös erreicht werden. Bei einer weiteren Variante besteht die Möglichkeit, dass die Regenbogenhaut mittels ein oder mehrerer in die Vorderkammer eingeführter sowie in Umfangsrichtung an der Regenbogenhaut im Abstand zueinander eingehängter Operationshaken zurückgezogen wird. Anschliessend wird der einzelne Operationshaken mit einem daran verschiebbar angeordneten Fixierelement an der äusseren Kontur des Auges positioniert und vorzugsweise abdichtend gehalten. Nach dem operativen Eingriff werden die eingehängten Operationshaken wieder von der Regenbogenhaut gelöst und aus der Vorderkammer des Auges herausgezogen.

Aus den Druckschriften (EP-A 0 653 197 und US-A 5,716,328) ist jeweils ein Operationshaken (RETRACTOR) für die Augenchirurgie bekannt, wobei der einzelne Operationshaken entweder mit einem einzigen oder zwei parallel zueiander angeordneten und je mit einem angeformten Einhängeteil versehenen Führungskörper zum Zurückziehen der Regenbogenhaut (IRIS) in die Vorderkammer des Auges einführbar ist und durch ein am Führungskörper in Längsrichtung verschiebbar angeordnetes Fixierelement im Bereich des Übergangs von der Hornhaut (CORNEA) zur Lederhaut (SKLERA) gehalten ist. Bei den bekannten Operationshaken hat der einzelne Führungskörper eine Länge von etwa 5 mm bis 8 mm sowie eine etwa 0,15 mm bis 0,20 mm Dicke und ist beispielsweise aus flexiblem Material, vorzugsweise aus flexiblem thermoplastischen Material hergestellt, wobei das mindestens an einem Ende angeformte Einhängeteil eine Länge von etwa 1,0 mm bis 1,5 mm aufweist.

Die Verwendung derartiger Operationshaken bei operativen Eingriffen im vorderen und hinteren Augenabschnitt hat sich bewährt, wobei jedoch die Operationshaken beim Einführen in die Vorderkammer des Auges sowie beim Einhängen an der Regenbogenhaut beziehungsweise beim Entfernen aus der Vorderkammer aufgrund der relativ kleinen Abmessungen für den Augenchirurgen (Ophthalmologen) nur sehr schwer erkennbar sind.

Die Aufgabe der vorliegenden Erfindung besteht darin, einen Operationshaken zum Zurückziehen der Regenbogenhaut so zu gestalten, dass dieser von dem Augenchirurgen während des operativen Eingriffs sowie beim anschliessenden Entfernen gut erkennbar und somit eine zuverlässige Überprüfbarkeit noch vorhandener Operationshaken nahezu ohne zusätzliche optische Hilfsmittel möglich ist.

Diese Aufgabe wird erfindungsgemäss dadurch gelöst, dass der Führungskörper ausgehend von dem in die Vorderkammer des Auges einführbaren hakenförmigen Einhängeteil in axialer Richtung bis mindestens zur Hälfte der gesamten Länge aus flexiblem und in Bezug auf die Farbe der Regenbogenhaut kontrastfarbenem polymeren Material hergestellt ist.

Mit dem erfindungsgemässen Operationshaken (Retractor) und der Verwendung desselben wird erreicht, dass die einzelnen in die Vorderkammer des Auges eingeführten und in Umfangsrichtung verteilt zueinander an der Regenbogenhaut eingehängten Operationshaken auch während des operativen Eingriffs für den Augenchirurgen sehr gut sichtbar und aufgrund dessen nach dem Eingriff wieder zuverlässig aus dem Auge entfernbar sind. Als besonders vorteilhaft hat sich herausgestellt, dass mit dem kontrastfarbenen sowie beim Einführen und beim Entfernen aus dem Auge optimal visuell erkennbaren Operationshaken eine optimale Handhabung gewährleistet ist.

Weitere Merkmale der Erfindung ergeben sich aus der Beschreibung in Verbindung mit der Zeichnung und den einzelnen Patentansprüchen.

Die Erfindung wird nachstehend anhand der Zeichnung beschrieben. Es zeigt:
- **Fig. 1**: ein schematisch und in grösserem Massstab dargestelltes Teilstück eines Auges mit einem in die Vorderkammer eingeführten Operationshaken zum Zurückziehen der Regenbogenhaut (Iris);
- **Fig.2**: das in Draufsicht dargestellte Teilstück des Auges mit der beispielsweise an zwei beliebig gewählten Stellen mittels eines ersten Operationshakens zurückgezogenen Regenbogenhaut;
- **Fig.3**: das in Draufsicht dargestellte Teilstück des Auges gemäss Fig.2 mit der mittels eines zweiten Operationshakens an beliebig gewählter Stelle zurückgezogenen Regenbogenhaut;
- **Fig.4**: den mit einem Führungskörper sowie daran angeordnetem Fixierelement versehenen und in Ansicht sowie grösserem Massstab dargestellten ersten Operationshaken;
- **Fig.5**: den in Draufsicht dargestellten ersten Operationshaken gemäss Fig.4;
- **Fig.6**: den gemäss der Linie VI-VI in Fig.5 im Profilquerschnitt kreisförmig ausgebildeten Führungskörper für den ersten Operationshaken gemäss Fig.4;
- **Fig. 6A**: den im Profilquerschnitt ellipsenförmig ausgebildeten Führungskörper für den ersten Operationshaken gemäss Fig.4;
- **Fig.7**: den in Ansicht und grösserem Massstab dargestellten zweiten Operationshaken mit zwei parallel zueinander angeordneten Führungskörpern mit daran angeordnetem Fixierelement;
- **Fig. 8**: den in Draufsicht dargestellten Operationshaken gemäss Fig.7; und
- **Fig.9**: die beiden gemäss der Linie IX-IX in Fig.8 im Schnitt dargestellten Führungskörper des zweiten Operationshakens.

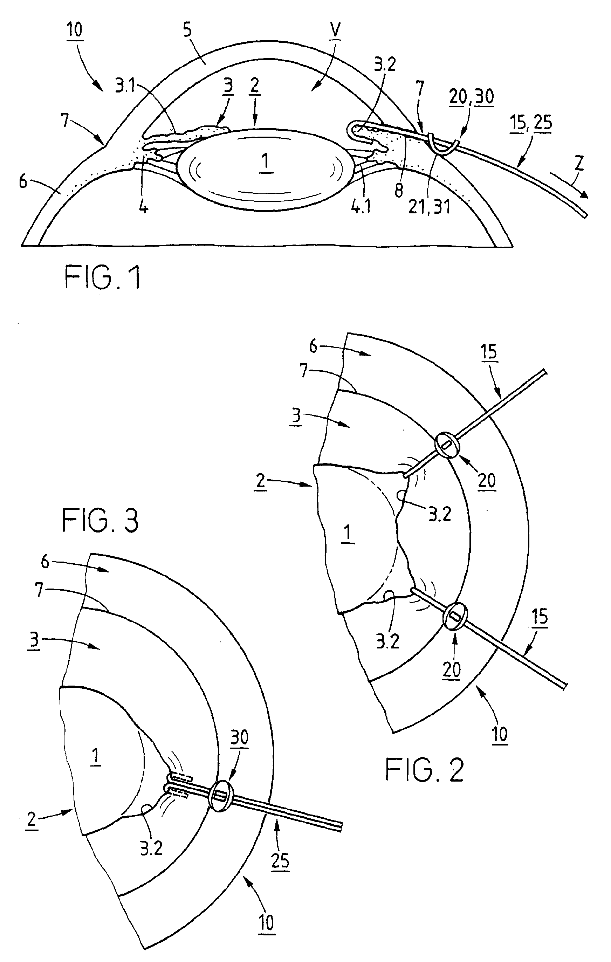

Zur Verdeutlichung der Erfindung ist in Fig.1 ein schematisch und als Horizontalschnitt dargestelltes Teilstück eines Auges 10 dargestellt und man erkennt die Vorderkammer V, die Linse 1 (OKULAR), die Pupille 2, die Regenbogenhaut 3 (IRIS) mit den beiden Teilstücken 3.1 und 3.2 sowie den Ziliarkörper 4 mit den Zonulafasern 4.1, die Hornhaut 5 (CORNEA) und die Lederhaut 6 (SKLERA) mit dem Übergang 7. Für einen operativen Eingriff im vorderen Augenabschnitt sowie bei Eingriffen im hinteren Augenabschnitt ist es erforderlich ein während des operativen Eingriffs möglichst grosses und unverändert bestehenbleibendes Sichtfeld für den Augenchirurgen (Ophthalmologen) zu gewährleisten.

Wie in Fig.1 schematisch dargestellt, wird zur Erreichung des Sichtfeldes beziehungsweise zur Vergrösserung der Pupille 2 in einer ersten Phase ein Operationshaken 15 oder 25 durch einen in der Hornhaut 5 vorgesehenen Einschnitt 8 in die Vorderkammer V des Auges 10 eingeführt. Mit dem an der Regenbogenhaut 3 eingehängten Operationshaken 15 oder 25 wird anschliessend das Teilstück oder der Bereich 3.2 der Regenbogenhaut 3 in Pfeilrichtung Z nach aussen gezogen. Der einzelne Operationshaken 15 oder 25 wird durch ein daran angeordnetes und in Längsrichtung desselben verschiebbares Fixierelement 20 oder 30 an dem Übergang 7 von der Hornhaut 5 zur Lederhaut 6 gehalten. Das Fixierelement 20 oder 30 ist vorzugsweise elastisch verformbar ausgebildet und mit einer bogenförmigen Anlagefläche 21 oder 31 etwa an den Übergang 7 anpassbar.

Fig.2 zeigt das in Draufsicht dargestellte Teilstück des Auges 10 und man erkennt den beispielsweise mittels zwei im Abstand zueinander angeordneter Operationshaken 15 zurückgezogenen Bereich 3.2 der Regenbogenhaut 3. Der einzelne Operationshaken 15 ist dabei mittels des jeweils daran angeordneten Fixierelements 20 an dem Übergang 7 von der Hornhaut 5 zur Lederhaut 6 gehalten.

Fig.3 zeigt als weiteres Ausführungsbeispiel das in Draufsicht dargestellte Teilstück des Auges 10 und man erkennt den mittels des einzelnen Operationshakens 25 zurückgezogenen Bereich 3.2 der Regenbogenhaut 3. Der Operationshaken 25 ist mittels des daran angeordneten Fixierelements 30 ebenfalls am Übergang 7 von der Hornhaut 5 zur Lederhaut 6 gehalten.

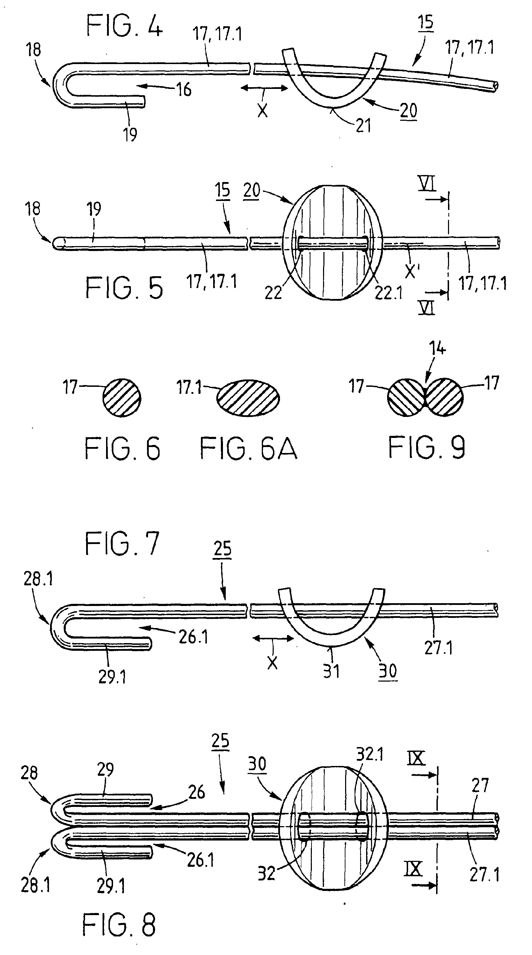

In Fig.4 ist der eine Operationshaken 15 in Ansicht und in Fig.5 in Draufsicht sowie in grösserem Massstab dargestellt. Der Operationshaken 15 hat einen länglichen Führungskörper 17, welcher mindestens an dem einen Ende ein einmal haarnadelförmig umgebogenes Einhängeteil 18 aufweist. Das Einhängeteil hat einen durch einen Spalt 16 parallel zum Führungskörper 17 angeordneten Schenkel 19. Das an dem Führungskörper 17 in Doppelpfeilrichtung X verschiebbar angeordnete Fixierelement 20 ist beispielsweise als kreisförmige Scheibe ausgebildet und mit zwei im Abstand zueinander angeordneten Bohrungen 22 und 22.1 versehen. Die beiden Bohrungen 22 und 22.1 sind derart zueinander angeordnet, dass das flexible Fixierelement 20 in weitgehend zusammengedrücktem Zustand auf den Führungskörper 17 geschoben und durch Loslassen an beliebiger Stelle daran positioniert werden kann. Das Fixierelement 20 bildet in aufgeschobenem Zustand eine bogenförmige Anlagefläche 21, welche in zurückgezogenem Zustand der Regenbogenhaut 3 am Übergang 7 des Auges 10 (Fig.1) anliegt.

Fig.6 zeigt den gemäss der Linie VI-VI in Fig.5 im Profilquerschnitt kreisförmig ausgebildeten Führungskörper 17. In Fig.6A ist als Variante ein Führungskörper 17.1 dargestellt, welcher abweichend von dem Führungskörper 17 beispielsweise im Profilquerschnitt ellipsenförmig ausgebildet ist.

Der vorstehend beschriebene Operationshaken 15 gemäss Fig.4 bis Fig.6 unterscheidet sich in technischer Hinsicht nicht von demjenigen, welcher bereits in dem EP-Patent 0 653 197 offenbart ist. Lediglich bezüglich der Farbauswahl des länglichen Führungskörpers 17 und/oder des Fixierelements 20 sind die anspruchsgemässen Merkmale vorliegender Erfindung zu beachten.

In Fig.7 ist der andere Operationshaken 25 in Ansicht und in Fig.8 in Draufsicht dargestellt. Der in grösserem Massstab dargestellte Operationshaken 25 hat zwei parallel zueinander angeordnete längliche Führungskörper 27 und 27.1, welche an den einander zugewandten Längsseiten in nicht dargestellter Weise, beispielsweise durch eine Klebverbindung 14 (Fig.9) oder dergleichen miteinander verbunden sind. An dem einen Ende des einzelnen Führungskörpers 27 und 27.1 ist jeweils ein einmal haarnadelförmig umgebogenes und jeweils mit einem Schenkel 29 beziehungsweise 29.1 versehenes Einhängeteil 28 und 28.1 angeformt. Die beiden Einhängeteile 28 und 28.1 mit den jeweils durch einen Spalt 26 und 26.1 parallel zum Führungskörper 27 und 27.1 angeordneten Schenkel 29 und 29.1 sind unter spitzem Winkel etwa A-förmig auseinanderlaufend zueinander angeordnet. Das an den beiden Führungskörpern 27 und 27.1 in Doppelpfeilrichtung X verschiebbar angeordnete Fixierelement 30 ist als kreisförmige Scheibe ausgebildet und mit zwei im Abstand zueinander angeordneten Bohrungen 32 und 32.1 versehen. Die Bohrungen 32 und 32.1 sind derart zueinander angeordnet, dass das flexible Fixierelement 30 in etwa zusammengedrücktem Zustand auf die beiden in Längsrichtung miteinander verbundenen Führungskörper 27 und 27.1 geschoben und durch Loslassen an beliebiger Stelle positioniert werden kann.

Der Operationshaken 25 gemäss Fig.7 bis 9 unterscheidet sich in technischer Hinsicht nicht von demjenigen, welcher bereits in dem US-Patent 5,716,328 offenbart ist. Lediglich bezüglich der Farbauswahl der beiden länglichen Führungskörper 27 und 27.1 und/oder des Fixierelements 30 sind die anspruchsgemässen Merkmale vorliegender Erfindung zu beachten.

Der Führungskörper 17 gemäss der Figuren 4 und 5 sowie die beiden Führungskörper 27 und 27.1 gemäss der Figuren 7 und 8 sind ausgehend von dem jeweiligen Einhängeteil 18,28 sowie 28.1 in axialer Richtung gesehen mindestens bis zur Hälfte der gesamten Länge vorzugsweise aber über die gesamte Länge aus einem flexiblen und in Bezug auf die Farbe der jeweiligen Regenbogenhaut 3 (Fig.2,3) kontrastfarbenen polymeren Material hergestellt. Der einzelne Führungskörper 17 oder 27 beziehungsweise 27.1 kann auch aus einem optisch signalfarbenen thermoplastischen Material, wie Polyamid oder dergleichen hergestellt werden.

Bei einer weiteren Variante besteht die Möglichkeit, dass der jeweils aus dem kontrastfarbenen polymeren Material oder aus thermoplastischem Material wie Polyamid oder dergleichen hergestellte Führungskörper 17 oder 27 und 27.1 eine in axialer Richtung orientierte und kontrastfarben eingefärbte, äussere kreisringförmige Partikelsektion, vorzugsweise zur Erreichung einer homogenen Einfärbung eine gedrängte kreisringförmige Gruppierung von Partikeln oder Teilchen (Farbteilchen) aufweist.

Weiterhin besteht die Möglichkeit, dass der aus polymerem Material oder aus thermoplastischem Material wie Polyamid oder dergleichen hergestellte flexible Führungskörper 17 oder 27 und 27.1 einen homogen und in bezug auf die Regenbogenhaut kontrastfarben eingefärbten Profilquerschnitt aufweist. Der flexible Führungskörper 17 oder 27 und 27.1 ist etwa in Form eines länglichen Filaments oder Fadens ausgebildet und hat einen Durchmesser von etwa 0,15 mm bis 0,20 mm.

Das einzelne an dem jeweiligen Führungskörper 17 oder 27 und 27.1 angeordnete und in axialer Richtung derselben verschiebbare Fixierelement 20 oder 30 ist vorzugsweise aus einer optisch signalfarben eingefärbten Kautschukmischung, beispielsweise aus einer Silikon-Kautschukmasse hergestellt.

Die entsprechend kontrastfarben eingefärbten Führungskörper 17 oder 27 beziehungsweise 27.1 sowie das daran angeordnete Fixierelement 20 oder 30 haben vorzugsweise zur Vermeidung einer unerwünschten Reflektion eine matte (glanzlos) äussere Oberfläche.

Nachstehend werden einige Anwendungsbeispiele der kontrastfarbenen Operationshaken 15 oder 25 in Abhängigkeit der Farbe der Regenbogenhaut 3 des Auges 10 angegeben:
**a)** Farbe der jeweiligen Regenbogenhaut 3: blau, blaugrau, grün, grüngrau oder einer Mischung davon;
   Farbe des Führungskörpers 17 oder 27 und 27.1: rot, signalrot, braun, schwarz oder tiefschwarz;
**b)** Farbe der jeweiligen Regenbogenhaut 3: braun oder dunkle Mischfarbe;
   Farbe des Führungskörpers 17 oder 27 und 27.1: gelb oder weiss.

Die vorstehend in Verbindung mit den einzelnen Anwendungsbeispielen erwähnten Farben der Führungskörper 17 oder 27 und 27.1 sowie der Fixierelemente 20 oder 30 in Verbindung mit den aufgeführten Farben der jeweiligen Regenbogenhaut 3 sind nicht auf die erwähnten Ausführungsbeispiele beschränkt.

Weitere zweckmässige Mischfarben für die aus einem flexiblen und in Bezug auf die Farbe der Regenbogenhaut kontrastfarbenen polymeren Material oder aus thermoplastischen Material wie Polyamid oder dergleichen hergestellten Führungskörper 17 oder 27 und 27.1 sowie für das aus einer Kautschukmischung hergestellte Fixierelement 20 oder 30 sind ebenfalls möglich.

## Patentansprüche

1. Operationshaken für die Augenchirurgie mit mindestens einem länglichen Führungskörper (17;27,27.1) und daran angeordnetem Einhängeteil (18;28,28.1) zum örtlichen Zurückziehen der Regenbogenhaut (3) sowie einem verschiebbar an dem Führungskörper angeordneten Fixierelement (20;30) zur Positionierung an der äusseren Kontur des Auges (10), **dadurch gekennzeichnet, dass** der Führungskörper (17;27,27.1) ausgehend von dem in die Vorderkammer (V) des Auges (10) einführbaren hakenförmigen Einhängeteil (18;28,28.1) in axialer Richtung bis mindestens zur Hälfte der gesamten Länge aus flexiblem und in Bezug auf die Farbe der Regenbogenhaut (3) kontrastfarbenem polymeren Material hergestellt ist.

2. Operationshaken nach Anspruch 1, **dadurch gekennzeichnet, dass** der Führungskörper (17;27,27.1) über die gesamte Länge aus flexiblem und optisch signalfarbenem polymeren Material hergestellt ist.

3. Operationshaken nach Anspruch 1, **dadurch gekennzeichnet, dass** der Führungskörper (17;27,27.1) über die gesamte Länge aus flexiblem und im Profilquerschnitt homogen kontrastfarben eingefärbtem polymeren Material hergestellt ist.

4. Operationshaken nach Anspruch 1, **dadurch gekennzeichnet, dass** der Führungskörper (17;27,27.1) im Profilquerschnitt kreisförmig ausgebildet ist und in Bezug auf die Farbe der Regenbogenhaut (3) aus homogen und kontrastfarben eingefärbtem flexiblem polymeren Material hergestellt ist.

5. Operationshaken nach Anspruch 1, **dadurch gekennzeichnet, dass** der Führungskörper (17;27,27.1) im Profilquerschnitt ellipsenförmig ausgebildet ist und in Bezug auf die Farbe der Regenbogenhaut (3) aus homogen und kontrastfarben eingefärbtem flexiblem polymeren Material hergestellt ist.

6. Operationshaken nach einem der Ansprüche 1 bis 5, **dadurch gekennzeichnet, dass** der Führungskörper (17;27,27.1) aus flexiblem und im Profilquerschnitt homogen rot oder signalrot eingefärbtem polymeren Material, beispielsweise aus thermoplastischem Material wie Polyamid oder dergleichen hergestellt ist.

7. Operationshaken nach einem der Ansprüche 1 bis 5, **dadurch gekennzeichnet, dass** der Führungskörper (17;27,27.1) aus flexiblem und im Profilquerschnitt homogen braun, schwarz oder tiefschwarz eingefärbtem polymeren Material, beispielsweise aus thermoplastischem Material wie Polyamid oder dergleichen hergestellt ist.

8. Operationshaken nach einem der Ansprüche 1 bis 5, **dadurch gekennzeichnet, dass** der Führungskörper (17;27,27.1) aus flexiblem und im Profilquerschnitt homogen gelb oder weiss eingefärbtem polymeren Material, beispielsweise aus thermoplastischem Material wie Polyamid oder dergleichen hergestellt ist.

9. Operationshaken nach Anspruch 1, **dadurch gekennzeichnet, dass** der aus flexiblem poymeren Material, beispielsweise aus thermoplastischem Material wie Polyamid oder dergleichen hergestellte Führungskörper (17;27,27.1) mindestens eine in axialer Richtung desselben orientierte äussere und im Profilquerschnitt kreisringförmig ausgebildete Partikelsektion aufweist, welche in Bezug auf die Farbe der Regenbogenhaut kontrastfarben eingefärbt ist.

10. Operationshaken nach Anspruch 9, **dadurch gekennzeichnet, dass** die äussere und im Profilquerschnitt kreisringförmig ausgebildete Partikelsektion des Führungskörpers (17;27,27.1) jeweils homogen rot oder signalrot eingefärbt ist.

11. Operationshaken nach Anspruch 9, **dadurch gekennzeichnet, dass** die äussere und im Profilquerschnitt kreisringförmig ausgebildete Partikelsektion des Führungskörpers (17;27,27.1) jeweils braun, schwarz oder tiefschwarz eingefärbt ist.

12. Operationshaken nach Anspruch 9, **dadurch gekennzeichnet, dass** die äussere und im Profilquerschnitt kreisringförmig ausgebildete Partikelsektion des Führungskörpers (17;27,27.1) jeweils homogen gelb oder weiss eingefärbt ist.

13. Operationshaken nach Anspruch 9, **dadurch gekennzeichnet, dass** die äussere und im Profilquerschnitt kreisringförmig ausgebildete Partikelsektion des Führungskörpers (17;27,27.1) homogen braun eingefärbt ist.

14. Operationshaken nach Anspruch 1, **dadurch gekennzeichnet, dass** das an dem Führungskörper (17;27,27.1) in axialer Richtung verschiebbar angeordnete Fixierelement (20;30) aus einer optisch signalfarben eingefärbten Kautschukmischung hergestellt ist.

15. Operationshaken nach Anspruch 9, **dadurch gekennzeichnet, dass** die optisch signalfarben eingefärbte Kautschukmischung für das Fixierelement (20;30) eine Silikon-Kautschukmasse ist.

16. Operationshaken nach Anspruch 1, **gekennzeichnet durch** die Verwendung eines mit mindestens einem Einhängeteil (18) versehenen Führungskörpers (17) und einem daran angeordneten Fixierelement (20), wobei mindestens der Führungskörper (17) aus flexiblem und in Bezug auf die Farbe der jeweiligen Regenbogenhaut (3) kontrastfarben eingefärbtem polymeren Material, beispielsweise aus thermoplastischem Material wie Polyamid oder dergleichen hergestellt ist.

17. Operationshaken nach Anspruch 1, **gekennzeichnet durch** die Verwendung zwei parallel zueinander angeordneter und jeweils mit mindestens einem Einhängeteil (28,28.1) versehener Führungskörper (27,27.1) sowie einem daran angeordneten Fixierelement (30), wobei mindestens die beiden Führungskörper (27,27.1) aus flexiblem und in Bezug auf die Farbe der jeweiligen Regenbogenhaut (3) kontrastfarben eingefärbtem polymeren Material, beispielsweise aus thermoplastischem Material wie Polyamid oder dergleichen hergestellt sind.

18. Operationshaken nach den Ansprüchen 16 und 17, **gekennzeichnet durch** die Verwendung eines rot oder signalrot eingefärbten Führungskörpers (17;27,27.1) für die Regenbogenhaut (3) des jeweiligen Auges (10) mit blauer, blaugrauer, grüner, grüngrauer Farbe oder einer Farbmischung davon.

19. Operationshaken nach den Ansprüchen 16 und 17, **gekennzeichnet durch** die Verwendung eines braun, schwarz oder tiefschwarz eingefärbten Führungskörpers (17;27,27.1) für die Regenbogenhaut (3) des jeweiligen Auges (10) mit blauer, blaugrauer, grüner, grüngrauer Farbe oder einer Farbmischung davon.

20. Operationshaken nach den Ansprüchen 16 und 17, **gekennzeichnet durch** die Verwendung eines gelb oder weiss eingefärbten Führungskörpers (17;27,27.1) für die Regenbogenhaut (3) des jeweiligen Auges (10) mit brauner oder einer dunklen Mischfarbe.
